# EUROPEAN PATENT SPECIFICATION

(11) **EP 3 064 175 B1**
(45) Date of publication and mention of the grant of the patent: **01.11.2023**
(21) Application number: 16156901.7
(22) Date of filing: 23.02.2016
(51) Int. Cl.: A61F 2/44, A61F 2/30

(54) **ORTHOPAEDIC IMPLANT WITH POROUS STRUCTURAL MEMBER**
WIRBELPROTHESE MIT PORÖSEM STRUKTURELEMENT
IMPLANT VERTÉBRAL AVEC ÉLÉMENT DE STRUCTURE POREUSE

(30) Priority: 03.03.2015 US 201514637142
(43) Date of publication of application: 07.09.2016
(73) Proprietor: SMed - TA/TD LLC, Columbia City, IN 46725 (US)
(72) Inventor: Nebosky, Paul S., Fort Wayne, IN 46807 (US); Stalcup, Gregory C., Columbia City, IN 46725 (US)
(74) Representative: Sawodny, Michael-Wolfgang

(56) References cited:
- US-A1- 2003 105 527
- US-A1- 2006 200 166
- US-A1- 2007 213 827
- US-A1- 2009 254 182
- US-A1- 2011 190 888
- US-A1- 2012 316 650

## Description

### BACKGROUND OF THE INVENTION

### 1. Field of the Invention

The present invention relates to orthopaedic devices, and, more particularly, to orthopaedic implants.

### 2. Description of the Related Art

Most orthopaedic implants are formed from a metallic material suitable for a given implant, such as a hip implant, knee implant, glenoid implant, etc. In the case of articulating joints, the implant may include a non-metallic load bearing surface, such as an ultra high molecular weight polyethylene (UHMWPE). The UHMWPE is attached to the metallic body of the implant, and provides the implant with good wear characteristics and low friction.

It is also known to provide an implant with a porous bony ingrowth surface. For example, a hip implant may include a porous surface on the stem which is intended to allow bony ingrowth of the proximal end of the femur bone. Such a porous surface may be in the form of a metal porous surface which is bonded, such as by heat sintering, to the stem of the implant. Examples of porous surfaces of this type include a woven mesh, a fiber mesh and particles. Knee implants are also known that include porous ingrowth surfaces that can bear load from surrounding anatomic structures.

Porous surfaces of the type described above which are used with implants are not typically part of a single structural member with two opposed, external porous surfaces. For example, in a knee implant, the distal surface of the implant can sit on the porous material that is slightly above the substrate material, but the porous material only typically has one external surface for tissue ingrowth. For hip implants, the porous ingrowth surface is usually provided as a coating on a structural component of the implant, such as the stem.

In some orthopaedic applications, such as spinal cages, it is beneficial to have a porous member that extends between two external, load bearing surfaces of the implant. In such arrangements, a cavity is typically formed between the two external surfaces of the implant and filled with a porous ingrowth material, which is typically a natural substance such as cancellous bone tissue. Such an implant is described in U.S. Patent Application No. 2002/0091447 to Shimp et al. One problem with the implant described by Shimp et al. is that harvesting sufficient cancellous bone tissue to fill the cavity is expensive, and host rejection issues can be a concern. Other similar implants that contemplate utilizing natural or synthetic materials are described in U.S. Patent Application Publication No. 2004/0210316 to King et al., and U.S. Patent No. 6,423,095 to Van Hoeck et al. In each of these described implants, the porous material held in the cavity is fairly isolated from bearing load from surrounding anatomic structures after implantation, with external surfaces that are either flush or below the most protruding external surface of the main implant body. This is intentional, as the materials placed in the cavity tend to have significantly lower strength than the implant body. However, isolating the porous ingrowth material from bearing loads from surrounding anatomic structures also decreases the amount of surface area the porous ingrowth material has in contact with the anatomic structures, which can slow down integration of the implant. In addition, the porous materials placed in the cavity are typically resorbable by the body and will not last throughout the life of the implant. From US 2003/010552 A1 an apparatus for facilitating fusion of adjacent vertebrae has made known including an implant body dimensioned for positioning within an intervertebral space between upper and lower vertebrae to maintain the vertebrae in desired spaced relation to facilitate fusion thereof. The implant body includes lower and upper surfaces for engaging the respective lower and upper vertebrae and first and second side all portions extending between the upper and lower surfaces. The first and second wall portions are substantially solid. At least one of the first and second side wall portions have a substantially narrow longitudinal slit defined therein arranged to enhance flexibility of the one side wall portion. Preferably, each of the first and second side wall portions includes a longitudinal slit. The implant body may define an internal chamber dimensioned for reception of bone growth inducing substances. The implant body includes an internal bore extending through the upper and lower surfaces for reception of bone growth inducing substances.
US 2006/0200166 A1 provides implants, instruments and methods for bone fusion procedures. The implants are particularly advantageous for use between opposing vertebral bodies to facilitate stabilization or arthrodesis of an intervertebral joint. The implants include, at least, a support component that provides structural support during fusion. The implants also include a growth component. A growth component provides an environment conducive to new bone growth between the bones being fused. Several configurations enhance fusion.

Problem in the art and what is needed in the art is an orthopaedic implant that can overcome the disadvantages of known devices.

### SUMMARY OF THE INVENTION

The problem is solved by an orthopaedic implant with the features of claim 1.

Advantageous embodiments are disclosed in the subclaims.

The present invention provides an orthopaedic implant with a porous load bearing member held within a surface-to-surface cavity formed in the implant's body that projects outwardly away from an exterior surface of the implant.

The invention is directed to an orthopaedic implant, comprising: an implant body having a first surface, a second surface opposite said first surface, and a cavity formed therein that extends through said first surface and said second surface, said implant body being substantially non-porous; and a load bearing member comprising a substantially porous material held within said cavity, said load bearing member having a first contact surface extending out of said cavity and being proud of a portion of said first surface, wherein said substantially non-porous implant body is a unibody and surrounds an entire periphery of at least a portion of the load bearing member and the load bearing member comprises a metal or a polymer, wherein said load bearing member comprises at least one of polyether ether ketone (PEEK), tantalum, and titanium.and/or has a total volume and said interconnecting pores in aggregate occupy at least 60%, preferably 80% of said total volume, and said load bearing member has a second contact surface opposite said first contact surface, said load bearing member having interconnecting pores extending from said first contact surface to said second contact surface, wherein said thickness of said first contact surface relative to said first surface is constant throughout said first contact surface.

An advantage of the present invention is that the porous load bearing member can bear load from anatomic structures during implantation while providing a surface for tissue ingrowth.

### BRIEF DESCRIPTION OF THE DRAWINGS

The above-mentioned and other features and advantages of this invention, and the manner of attaining them, will become more apparent and the invention will be better understood by reference to the following description of embodiments of the invention taken in conjunction with the accompanying drawings, wherein:
Fig. 1 is a perspective view of an embodiment of a solid component of a device formed according to the present invention;
Fig. 2 is a perspective view of an embodiment of a porous component of a device formed according to the present invention;
Fig. 3 is a perspective view of a device created from the solid component shown in Fig. 1 and the porous component shown in Fig. 2;
Fig. 4 is a cross-sectional view of a single, continuous layer with porous and solid regions;
Fig. 5 is a perspective view of an embodiment of a spinal cage with windows;
Fig. 6 is a cross-sectional view of the spinal cage shown in Fig. 5 taken along line 6-6;
Fig. 7 is a perspective view of an embodiment of a spinal cage with a ledge or groove;
Fig 8 is a cross-sectional view of the spinal cage shown in Fig. 7 taken along line 8-8;
Fig. 9 is a perspective view of an embodiment of a spinal cage with a two-part solid component that is assembled to contain the porous material;
Fig. 10 is a cross-sectional view of the spinal cage shown in Fig. 9 taken along line 10-10;
Fig. 11 is a perspective view of an embodiment of a spinal cage with laminates perpendicular to an axis of the spinal cage;
Fig. 12 is a perspective view of an embodiment of a spinal cage with laminates parallel to an axis of the spinal cage;
Fig. 13 is a perspective view of an embodiment of a spinal cage with laminates at an angle to an axis of the spinal cage;
Fig. 14 is a perspective view of an embodiment of a spinal cage;
Fig. 15 is a perspective view of another embodiment of a spinal cage;
Fig. 16 is a perspective view of yet another embodiment of a spinal cage;
Fig. 17 is a perspective view of yet another embodiment of a spinal cage;
Fig. 18 is a sectional view of an implant with features for the delivery of therapeutic agents;
Fig. 19 is a sectional view of a tapered implant;
Fig. 20 is a sectional view of another tapered implant;
Fig. 21 is a sectional view of yet another tapered implant;
Fig. 22 is a sectional view of yet another tapered implant;
Fig. 23 is a sectional view of yet another tapered implant;
Fig. 24 is a perspective view of an implant showing teeth that mate with surrounding bone;
Fig. 25 is a side view of the implant shown in Fig. 24;
Fig. 26 is a spinal fusion device;
Fig. 27 is a perspective view of another embodiment of an orthopaedic implant according to the present invention;
Fig. 28 is a side view of the orthopaedic implant shown in Fig. 27;
Fig. 29 is a front view of the orthopaedic implant shown in Figs. 27-28;
Fig. 30 is a perspective view of yet another embodiment of an orthopaedic implant according to the present invention;
Fig. 31 is a side view of the orthopaedic implant shown in Fig. 30;
Fig. 32 is a perspective view of yet another embodiment of an orthopaedic implant according to the present invention;
Fig. 33 is a front view of the orthopaedic implant shown in Fig. 32;
Fig. 34 is a side view of the orthopaedic implant shown in Figs. 32-33;
Fig. 35 is a side view of the orthopaedic implant shown in Figs. 32-34 including an ingrowth material;
Fig. 36 is a perspective view of yet another embodiment of an orthopaedic implant according to the present invention;
Fig. 37 is a side view of the orthopaedic implant shown in Fig. 36; and
Fig. 38 is a side view of the orthopaedic implant shown in Figs. 36-37 including an ingrowth material.

The exemplifications set out herein illustrate embodiments of the invention, and such exemplifications are not to be construed as limiting the scope of the invention in any manner.

### DETAILED DESCRIPTION OF THE INVENTION

### I. Porous spinal devices - laminate designs

Herein described is a laminate method for a spinal implant or implant component, including manufacturing methods for sheet creation, bonding/assembly methods, and ways of creating tapers. Further described is the delivery of therapeutic agents through a spinal device.

These methods do not form part of the claimed invention.

### A. Materials

Material options for the spinal device include the following: implantable polymers (such as PEEK, PMMA), implantable reinforced polymers (such as carbon-fiber reinforced PEEK), implantable metals (such as titanium, titanium alloy), and implantable ceramics (such as hydroxyapatite, alumina). One or more of these materials can be combined in a given device.

### B. Overall Design

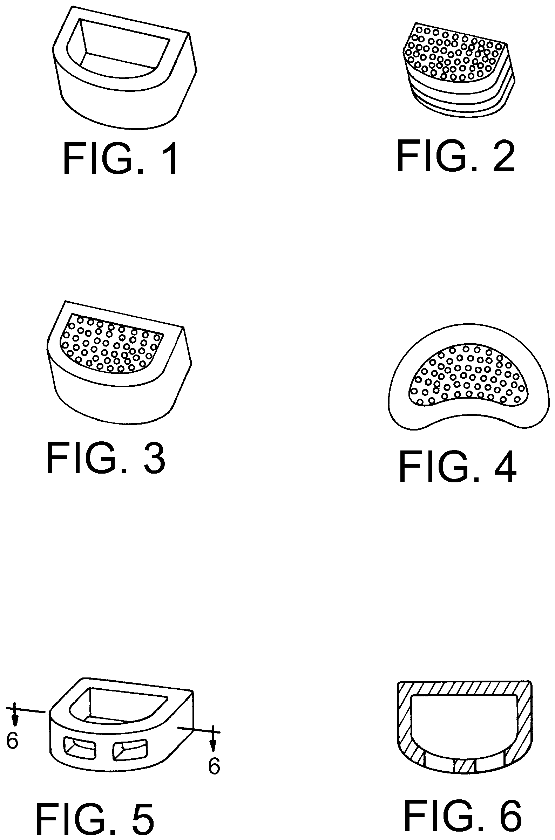

With regard to the overall design, the implant can include entirely porous material or one or more porous regions and one or more solid regions. Additionally, an entirely porous device can be created to mate with existing solid devices (See Figs. 1-3).

The porous region is created by stacking layers of material with interconnecting holes/geometry (hereafter referred to as holes).

The solid region can be formed by traditional techniques such as injection molding or machining or by bonding solid sheets together. The later method allows the solid and porous regions to be created from continuous sheets (See Fig. 4).

The holes in the sheets can be created by, for example, laser cutting, punching, etching, electrical discharge machining, plasma etching, electroforming, electron beam machining, water jet cutting, stamping, or machining. For polymer based materials, they can be created as the sheets are created by, for example, extruding, injection molding, or hot stamping.

Attachment of the sheets to each other can be achieved by any number of ways, including the following:
1. Heat. Heat can be generated by several ways:
   a. Ultrasonic welding - use ultrasonic waves to create heat at the interface of layers.
   b. Heat staking - use a heated tool to cause melting between the layers
   c. Vibratory welding
   d. Laser welding
   e. Convection - use an oven to create heat to cause bonding
   f. Intermediary layer - for example, use a material that can absorb energy waves that pass through the polymer (for example PEEK) without causing damage. The absorbed energy will cause localized heating. An example of such a coating is Clearweld by Gentex^{®} Corporation. The laser waves that Clearweld absorbs pass through the PEEK without causing damage, allowing the layers to be melted together without large scale damage to the PEEK.
2. Chemical.
   a. Adhesives - a secondary material (such as adhesive) can be used to bond the material.
   b. Solvent bonding - a material in which the polymer or reinforced polymer is soluble can be applied to the sheet surfaces allowing multiple surfaces to be bonded to one another.
   c. Overmolding - overmolding of the polymer or reinforced polymer can provide a chemical bonding
3. Mechanical.
   a. Overmolding - overmolding of a polymer or reinforced polymer can create a mechanical lock between components on a micro or macro scale (microscale - the molded material locks with surface asperities of the existing material. Macroscale - features such as tongue-groove connections or undercuts). The overmolded material can be a separate component from the layers or one layer can be overmolded onto another layer.
   b. Features are provided within the layers or by a separate component which provides a mechanical lock - e.g. A pin, snap lock connection, dove-tail, tongue-groove, rivet, screw and / or melting tabs to create a mechanical lock. For example, one or more rivets can connect all layers of a porous implant together. These connection features can be made of any implantable material including, but not limited to, titanium, titanium alloy, PEEK, and/or other implantable polymers. These features can also be used as radiopaque markers as is described below.
   c. Some adhesives provide a mechanical bond in addition to or instead of a chemical bond.
4. Combinations of any/all of the above methods.

If the porous and solid regions are created separately (as in Figs. 1-3), it may be desirable to bond the two together. There are several methods of achieving this bond:
1. Heat. Heat can be generated by several ways:
   a. Ultrasonic welding - use ultrasonic waves to create heat at the interface of layers.
   b. Heat staking - use a heated tool to cause melting between the layers
   c. Vibratory welding
   d. Laser welding
   e. Convection - use an oven to create heat to cause bonding
   f. Intermediary layer - for example, use a material that can absorb energy waves that pass through the polymer (for example PEEK) without causing damage. The absorbed energy will cause localized heating. An example of such a coating is Clearweld by Gentex^{®} Corporation. The laser waves that Clearweld absorbs pass through the PEEK without causing damage, allowing the layers to be melted together without large scale damage to the PEEK.
2. Chemical.
   a. Adhesives - a secondary material (such as adhesive) can be used to bond the material.
   b. Solvent bonding - a material in which the polymer or reinforced polymer is soluble can be applied to the sheet surfaces allowing multiple surfaces to be bonded to one another.
   c. Overmolding - overmolding of the polymer or reinforced polymer can provide a chemical bonding
3. Mechanical.
   a. Overmolding - overmolding of a polymer or reinforced polymer can create a mechanical lock between components on a micro or macro scale (microscale - the molded material locks with surface asperities of the existing material. Macroscale - features such as tongue-groove connections or undercuts). The overmolded material can be a separate component from the layers or one layer can be overmolded onto another layer.
   b. Features are provided within the layers or by a separate component which provides a mechanical lock - e.g. A pin, snap lock connection, dove-tail, tongue-groove, rivet, and / or melting tabs to create a mechanical lock. For example, the porous material can attach to the windows that are typical in spinal cages or to a groove or ledge is created along the interior edge of the solid ring (see Figs. 5-10). These connection features can be made of any implantable material including, but not limited to, titanium, titanium alloy, PEEK, and/or other implantable polymers. These features can also be used as radiopaque markers as is discussed later in this disclosure.
   c. Some adhesives provide a mechanical bond in addition to or instead of a chemical bond.
4. Combinations of any/all of the above methods.

Assembly of layer to layer or one component to another (for example a porous component to a solid component) can be aided by such ways as surface modifications to improve adhesive or solvent bonding or roughened surfaces.

Figs. 5-6 illustrate a spinal cage showing windows (a cross section view is shown at the right). This is an example of a type of feature onto which the porous component can be bonded.

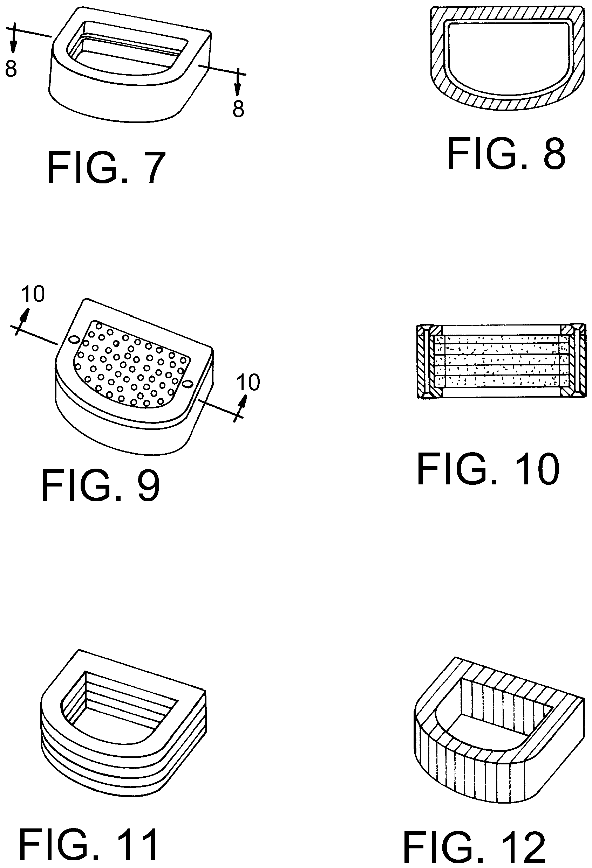

Figs. 7-8 illustrate a spinal cage showing a ledge or groove (a cross section view is shown at the right). This is an example of a type of feature onto which the porous component can be bonded.

Figs. 9-10 illustrate a spinal cage showing a two-part solid component that is assembled to contain the porous material. In this example mechanical means (screw/rivet) are used in conjunction with an adhesive bond. Adhesive ways alone, mechanical ways alone or any of the other manufacturing methods discussed in this disclosure are also options.

Figs. 11-13 illustrate a spinal cages showing laminates perpendicular, parallel, and at an angle to the axis of the implant.

The laminate portion of the implant can have layers oriented in any direction. For example, the layers can be perpendicular, parallel, or at an angle to the axis of the implant (See Figs. 11-13). This angle need not be constant within an implant.

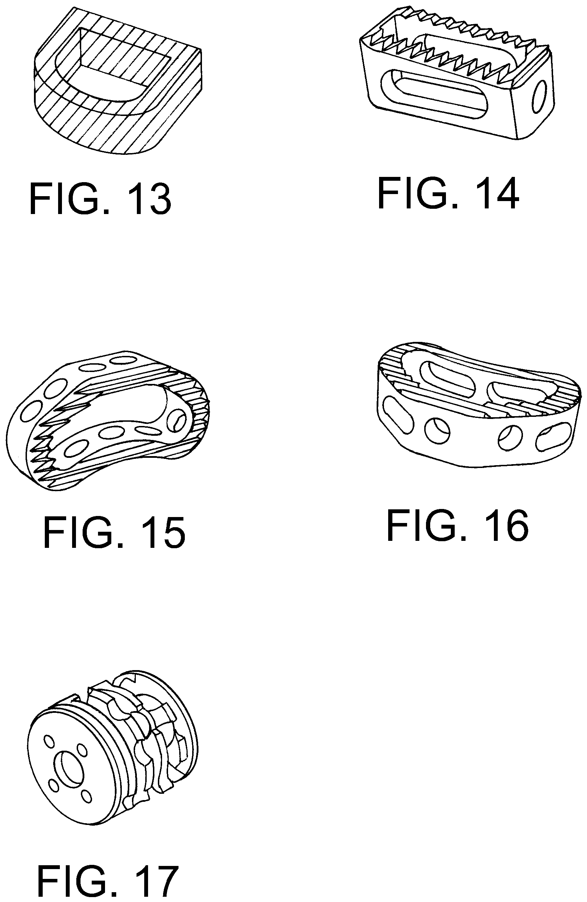

The overall shape of the implant can be of any typical existing type, such as ALIF, TLIF, PLIF, and standard round cages (see Figs. 14-17)

### C. Delivery of therapeutic agent.

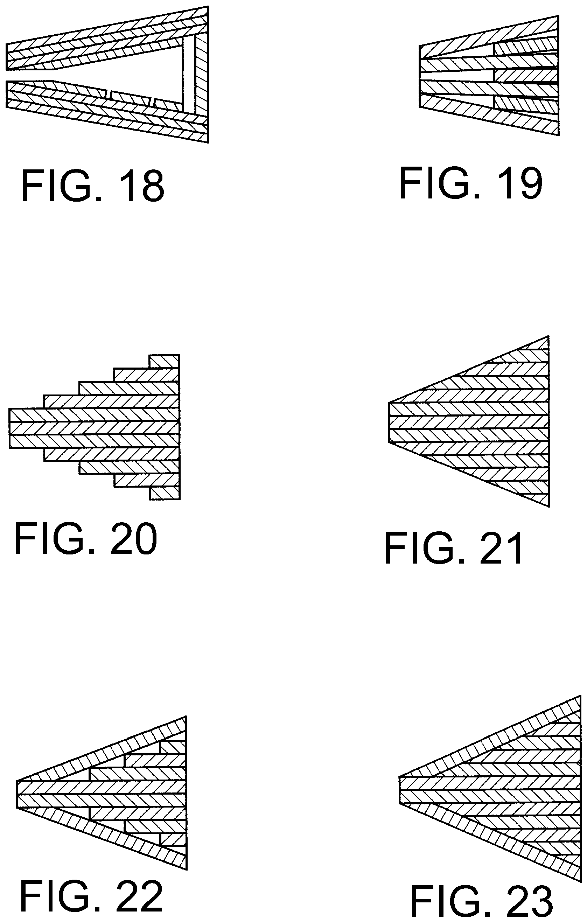

This device can be used to deliver therapeutic agents directly to the tissue surrounding the implant (See Fig. 18). Some examples of situations in which this would be desired: delivery of oncology treatments to cancerous tissue or tissue surrounding cancerous tissue; delivery of agents (such as BMP, hydroxyapatite slurry, and/or platelets) to encourage/enhance bone growth to promote faster and better fusion; and delivery of analgesic agents to reduce pain. This list is not exhaustive.

Fig. 18 illustrates a sectioned, side-view of an implant with features for the delivery of therapeutic agents.

The implant can include a reservoir for delivery of the therapeutic agent over an extended period of time. Openings leading from the reservoir to the porous material allow for controlled release of the therapeutic agents at a desired rate. The reservoir can be refilled at any time before, during, or after the surgery.

If immediate delivery of the therapeutic agents to the surrounding tissue is all that is required (not extended time release), the design need not include a reservoir. In this case, the therapeutic agents can be directly routed from the implant access to the porous material via channels. However, a reservoir can be included in an immediate delivery design; the openings in the reservoir would be sized to allow for immediate release of the therapeutic agent rather than a slower, long-term delivery.

The access in the implant (see Fig. 18) can mate with an insertion of a delivery tool (such as a needle) or a device (or catheter leading to a device) to allow for remote filling of the reservoir (such as by way of a subcutaneous port or external pain-pump).

In order to allow and promote bone growth through the implant from one vertebra to the other, openings run from the superior to the inferior portion of the implant and be appropriately sized to allow for bone ingrowth (See Fig. 18).

### D. Anterior-Posterior taper

Some implants are tapered to mate with the natural anterior-posterior taper that exists between vertebrae. If a solid portion exists, this taper can be created by traditional machining and/or molding techniques. In the porous region, there are several ways of creating this taper, including the following:
a. If the design includes a reservoir, the reservoir itself can be tapered. The porous ingrowth layers can be of uniform thickness and layered outside of the reservoir (as indicated in Fig. 18).
b. A wedge-shaped piece or pieces can create the taper with the ingrowth layers stacked on the wedge(s). This is essentially the same design as shown in Fig. 20 without the reservoir, access and holes for the therapeutic agent delivery. To allow and promote bone growth through the implant from one vertebra to the other, openings run from the superior to the inferior portion of the implant and be appropriately sized to allow for bone ingrowth (See Fig. 18).
c. Shorter layers can be stacked with larger layers to create an overall taper as in Fig. 19.
d. Layers of varying lengths can be sacked to create a stepped taper as in Fig. 20.
e. Similar to the technique in (d), layers of varying length can be stacked. A smooth taper can be created by using layers that are tapered prior to stacking or the smooth taper can be created, by such ways as machining or hot forming, after the layers are stacked. The second of these would involve first creating a part like that in (d), then removing material to create the smooth taper shown in Fig. 21.
f. Another way of creating a smooth surface on a stepped taper is to have one or more outer layers which are parallel to the taper face, as shown in Fig. 22.
g. The design in (f) does not allow for a large amount of contact area between the outer layer of the taper and the corners of the stepped layer. One way of providing increased contact area (which can provide increased strength) is to taper the stepped layers as in Fig. 21 before adding the outer layer(s) that are parallel to the face of the taper. An example of this is shown in Fig. 23.

### E. Interface with bone

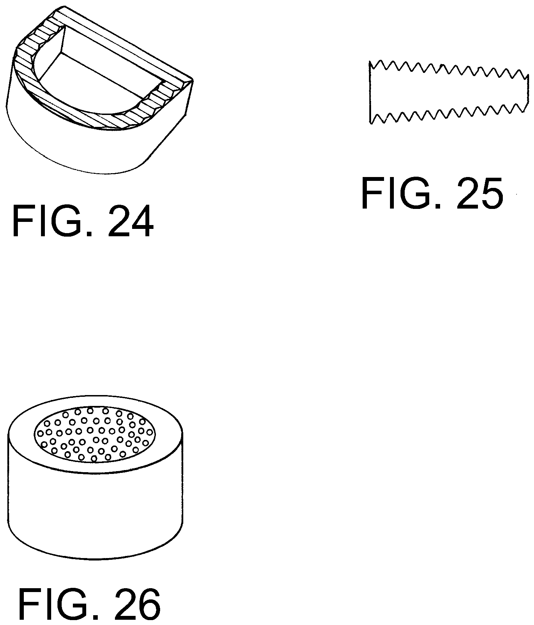

It is often desirable to have an implant-bone interface with relative high friction. Traditionally, this is achieved by such ways as a roughened implant surface, teeth (See Figs. 24-25), spikes, or hooks.

In a laminate implant, there are several options for creating such features. These options include the following:
a. Form features prior to bonding laminate sheets: Form teeth or other "rough" features into the outermost layers of the implant prior to bonding them to the other sheets. These teeth can be created by several ways:
   i. Form material - for example: heat forming, cold forming.
   ii. Remove material - for example: machining, laser cutting, chemical etching.
   iii. Add material - attach material to create the features by, for example, insert molding, mechanical attachment, adhesive bonding, laser welding, solvent bonding.
b. Form features after bonding laminate sheets: Form the rough surface features on the faces of the implant after the sheets have been bonded. These features can be formed by the same ways as listed in (a).
c. Secondary feature (such as hooks, spikes, etc) protruding from the implant into the bone. This feature can be attached by, for example, insert molding, mechanical attachment, adhesive bonding, laser welding, or solvent bonding.

Figs. 24-25 illustrate an implant showing teeth that mate with the surrounding bone.

### F. Interface with instruments

To aid in insertion of the implant into position in the body, it is often necessary to attach the implant to instrumentation. The material near the interface of the instrument and implant can often see additional stress. In a partially or fully laminate implant, it may be necessary to provide additional support in the region of this interface. This can be achieved by a number of ways, including: designing the instrument to reduce stresses and/or strengthening the implant in the region of the interface. For example, in the case of an instrument that contains a male thread which mates with a female thread in the implant, the implant can be strengthened by adding metal, solid polymer, or reinforced polymer in the region of the female thread. In machine design, thread inserts are frequently used to repair damaged threads. In this case, thread inserts can be used to strengthen the implant at the interface with the instrument(s).

### G. Radiopaque markers

When a radiolucent material, such as unfilled PEEK, is used, it is sometimes desirable to have the ability to see some or all of that implant on a diagnostic tool such as x-ray without the white-out problems of solid metal. For example, the surgeon may use such markers to determine the orientation and position of the implant to ensure proper placement during surgery. Radiopaque markers can provide this ability. The opacity and/or amount of radiopaque material can be controlled so that the marker does not prevent evaluation of the tissue near the implant by x-ray or other diagnostic ways. Material options include, but are not limited to, the following:
a. Implantable metals (stainless steel, titanium, or titanium alloys for example).
b. Barium sulfate filled PEEK.
c. Carbon filled PEEK.
d. Other polymers with radiopaque material (such as barium sulfate or zirconium dioxide).

Examples of the marker design include one or more of the following:
a. One or more radiopaque pins.
b. Assembly features such as rivets or pins.
c. Coating a portion of the device with a radiopaque material. Examples of methods for creating a radiopaque coating include, but are not limited to, the following:
   i. Using chemical vapor deposition to deposit a layer of titanium onto the polymer.
   ii. Using a radiopaque ink such as Radiopaque^{™} ink (developed by CI Medical).
d. One or more of the laminate layers being radiopaque. Examples of methods to make the layer(s) radiopaque include, but are not limited to, the following:
   i. Making the layer from an implantable metal (such as tantalum, titanium, titanium alloy, cobalt chrome, or stainless steel).
   ii. Using a barium sulfate filled polymer to create the layer.
   iii. Coating the layer with a radiopaque material - for example, using chemical vapor deposition to deposit a layer of titanium onto the surface of one or more layers.
e. A slightly radiopaque porous material. This can be achieved, for example, by using a polymer with barium sulfate.

### II. Porous polymer spinal fusion devices

The key to the success of a spinal fusion surgery is the formation of good bone growth between the vertebrae that are being fused. Evaluation of this bone growth is, thus, critical to determining the progress and eventual success of the surgery.

Existing porous spinal cages are made of biocompatible metals. Due to the density of these metals, the implants made post-operative examination of the tissue surrounding the implant difficult.

Several current devices are now made from solid biocompatible polymers such as PEEK. PEEK is a relatively radiolucent material. While this addresses the issue of radiopacity for solid fusion devices, it is often desired to encourage more rapid bone growth between the two vertebrae.

One solution for this problem is implants made from porous biocompatible polymers, such as PEEK or reinforced porous PEEK.

### A. Overall design

Such implants can be entirely porous or have a mix of porous and solid polymer. For example, a solid ring of material can surround a porous core (See Fig. 26).

Fig. 26 illustrates a spinal fusion device with solid region (Region 1) and porous region (Region 2)

One embodiment of the design is a porous center component that mates with existing solid, ring-like devices. This device could be assembled with the solid device in a manufacturing setting or in the operating room.

If a solid region/component exists, the porous and solid regions may need, but do not necessarily need, to be attached to one another. Examples of methods that can be used to attach the porous and solid material are:
a. Mechanical features - snap-fit connections, 'dove-tail' types of connections.
b. Adhesive bonding.
c. Solvent bonding.
d. Heat applied by, for example, laser, ultrasonic or vibratory welding, convection heating, heat staking.

### B. Material

a. Method of creating porosity
   i. Laminate design - bonding sheets of material which contain holes.
   ii. Foaming methods.
   iii. Bond 'beads' of polymer - bead of any shape can be bonded together (via, for example, heating, adhesive bonding, or solvent bonding) to create a porous structure.
   iv. Mix of polymer and dissolvable material.
      1. One method involves creating a mixture of powdered implantable material (e.g. PEEK) and a powder (e.g. salt) that is soluble in something in which the implantable material is not soluble (such as water, isopropyl alcohol for the PEEK example). The mixture is then heated to bond the implantable particles together. Pressure can also be applied to aid in the bonding of particle to particle. Heat can be created by convection or other ways (such as coating the powder with a material that absorbs a given range of energy waves - such as laser waves - and causes heating. E.g. Clearweld coating by Gentex^{®} Corporation). Finally, dissolve away the filler to create the porous implantable material. This method can create net shape parts or raw material shapes from which individual parts can be created.
      2. Another method involves mixing an implantable polymer with a dissolvable material such as described above. The mixture is then pelletized and then injection molded to an intermediary or the final part shape. The filler is dissolved away to create the porous implantable polymer.
b. Reinforcement - If improved mechanical properties are desired, various reinforcing materials can be used. For example, carbon fiber or barium sulfate can be used.

### C. Radiopaque markers

It is sometimes desirable to have the ability to see some of the implant on a diagnostic tool such as an x-ray without the white-out problems of solid metal. For example, the surgeon may use such markers to determine the orientation and position of the implant to ensure proper placement during surgery. Radiopaque markers can provide this ability. The opacity and/or amount of radiopaque material can be controlled so that the marker does not prevent evaluation of the tissue near the implant by x-ray or other diagnostic ways. Material options include, but are not limited to, the following:
a. Implantable metals (stainless steel, titanium, or titanium alloys for example).
b. Barium sulfate filled PEEK.
c. Carbon filled PEEK.
d. Other polymers with radiopaque material (such as barium sulfate or zirconium dioxide).

Examples of the marker design include one or more of the following:
a. One or more radiopaque pins.
b. Coating a portion of the device with a radiopaque material. Examples of methods for creating a radiopaque coating include, but are not limited to, the following:
   i. Using chemical vapor deposition to deposit a layer of titanium onto the polymer.
   ii. Using a radiopaque ink such as Radiopaque^{™} ink (developed by CI Medical).
c. A slightly radiopaque porous material. This can be achieved, for example, by using a polymer with barium sulfate.

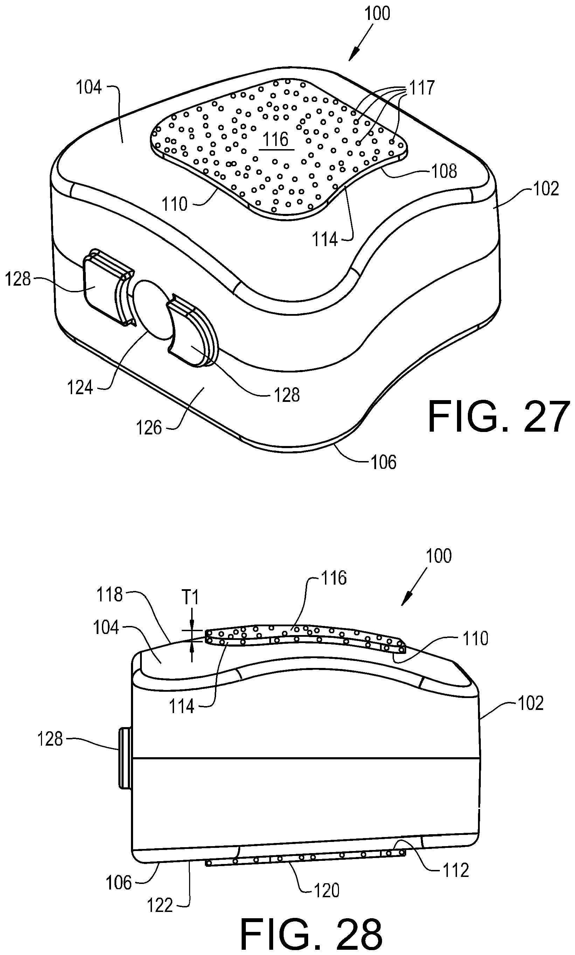

Referring now to Figs. 27-29, an embodiment of an orthopaedic implant 100 according to the present invention is shown that includes an implant body 102 formed from a substantially non-porous material having a first surface 104 and a second surface 106 opposite the first surface 104. As used herein, "substantially non-porous" indicates a porosity of 5% or less, so that the implant body 102 is mostly solid. The implant body 102 can be formed from a variety of different materials that are biocompatible and commonly used to form orthopaedic implants, including polyether ether ketone (PEEK), other polyaryl ether ketones (PAEKs), titanium, stainless steel, cobalt chrome, ultra-high molecular weight polyethylene (UHMWPE), or any previously described material. It should be appreciated that these materials are exemplary only and other biocompatible materials could be used to form the implant body. As shown in Figs. 27-29, the implant body 102 is formed in the shape of a cervical cage for spinal applications, but other shapes can also be used, as shown further herein. The first surface 104 and second surface 106 can be curved, as shown, or can be formed as planar surfaces that are substantially flat. Alternatively, one of the surfaces 104, 106 can be formed as a surface with one or more curvatures while the other surface is planar.

A cavity 108 is formed in the implant body 102 extending through the first surface 104 and second surface 106 to form a continuous cavity 108 through the implant body 102. The cavity 108 has a first cavity entrance 110 formed through the first surface 104 and a second cavity entrance 112 (shown in Fig. 28) formed through the second surface 106. One or both of the cavity entrances 110, 112 can be concentrically formed through their respective surface 104, 106 so that the cavity entrances 110, 112 have a perimeter shape that approximately matches a perimeter shape of their respective surface 104, 106, with the cavity entrances 110, 112 having a smaller perimeter than their respective surfaces 104, 106. The cavity 108 can be formed to have a constant or varying shape throughout.

A load bearing member 114 comprising a substantially porous material having a first contact surface 116 is held within the cavity 108 that is formed within the implant body 102. As used herein, "substantially porous" indicates a porosity of at least 20%, but can be significantly higher. For example, the load bearing member 114 can have a total volume, that is the entire volume occupied by the load bearing member 114, of which 60% or more is defined by pores 117 formed in the load bearing member 114. In other words, 40% of the total volume of the load bearing member 114 can be occupied by structural material forming the load bearing member 114 while 60% of the total volume is occupied by empty spaced defined by the pores 117, in aggregate. If an extremely porous material is used to form the load bearing member 114, the pores 117, in aggregate, can occupy 80% or more of the total volume of the load bearing member 114. If desired, one or more therapeutic agents can be held within some or all of the pores 117 for elution into surrounding anatomic features after implantation of the orthopaedic implant 100 to increase the efficacy of the surgical procedure. A non-exhaustive list of possible therapeutic agents that can be provided in the pores 117 includes various growth factors, bone morphogenetic factors, bone morphogenetic proteins, anti-microbial agents, antiinflammatories, anti-coagulants, painkillers, cytotoxic substances, stem cells, and any other substance, known or unknown, that is desirable to elute from the orthopaedic implant 100 following implantation. The material(s) used to form the load bearing member 114 should, like the implant body 102, be biocompatible so that the orthopaedic implant 100 is suitable for implantation at an anatomical site within a patient. It is also useful if the load bearing member 114 is formed from one or more materials that are non-resorbable, i.e., the material of the load bearing member 114 can maintain at least 90% of its original mass after being implanted in a living patient for at least a year. Examples of such materials are PEEK, tantalum, and titanium, but other porous materials are also contemplated as being used. The load bearing member 114 can comprise either a synthetic material, such as those previously described, or one or more naturally derived materials, such as a bone graft. The naturally derived material can also be, for example, cells or tissues harvested from the patient or a different organism, scaffolds created using collagen or other biomaterials, etc. It is useful, but not required, for the load bearing member 114 to substantially fill the cavity 108 so that at least 90% of the empty space in the implant body 102 defined by the cavity 108 is filled by the bearing member 114. Such filling of the cavity 108 by the load bearing member 114 makes it easier to hold the load bearing member 114 within the cavity 108 during implantation.

The first surface 104 defines a first peak 118, which is a point on the first surface 104 that has a maximum height, relative to a ground surface, when the second surface 106 of the implant body 102 is laid on the ground surface. The first peak 118 of implant body 102 is best shown in Fig. 28, where it can be seen that the first peak 118 is adjacent to the first cavity entrance 110. With further reference to Fig. 28, it can be seen that the first contact surface 116 of the load bearing member 114 extends out of the cavity 108 past the first cavity entrance 110 so that the first contact surface 116 extends past the first peak 118, i.e., the first contact surface 116 is proud of the first surface 104. In this sense, the first contact surface 116 defines a thickness T1 that extends past and projects from the first surface 104, which can be either constant or varying throughout the first contact surface 116. By extending the first contact surface 116 past the first peak 118 of the first surface 104, the first contact surface 116 can be placed in contact with an anatomic structure, such as a vertebrae, during implantation while isolating the first surface 104 from contact with the anatomic structure. Once implanted, the porous load bearing member 114 can then bear load from the anatomic structure while allowing for ingrowth of tissue into the load bearing member 114 through the pores 117.

Due to the varying shapes of anatomic structures and desired load bearing characteristics, the first contact surface 116 can be a curved surface or a planar surface. The relative sizing between the first surface 104 and the first contact surface 116 can also be adjusted, as desired, to balance the load bearing characteristics of the load bearing member 114. As can be seen, the first contact surface 116 defines a contact surface area and the first surface 104 defines a first surface area, with the contact surface area and first surface area together defining a top surface area of the orthopaedic implant 100. The relative percentage of the top surface area that the contact surface area makes up can be altered to give varying amount of contact surface for anatomic structures during implantation. It is contemplated that the contact surface area can be 40 to 90% of the total surface area when a large contact surface 116 is desired, or less than 40% of the total surface area when a smaller contact surface 116 is desired. It should be understood that the term "top surface area" is used for convenience of description only and not to limit the scope of the present invention.

Optionally, the load bearing member 114 can have a second contact surface 120 extending out of the cavity 108 past the second cavity entrance 112 so that it extends past a second peak 122 of the second surface 106 of the implant body 102. The second peak 122 of the second surface 106 is analogous to the first peak 118 of the first surface 104, with the key difference being that the second peak 122 defines a maximum height of the second surface 106 relative to a ground surface when the first surface 104 is laid on the ground surface. The second contact surface 120 can be configured and altered similarly to the first contact surface 116 so that the second contact surface 120 can be in contact with an anatomic structure following implantation. The second contact surface 120 can be a mirror image of the first contact surface 116 or a different configuration, depending on the desired load bearing characteristics of the load bearing member 114 caused by loads bearing on the first and second contact surfaces 116, 120 from surrounding anatomic structures. It can be useful if the pores 117 of the load bearing member 114 interconnect from the first contact surface 116 to the second contact surface 120 so that a travel path through the entirety of the load bearing member 114 can be formed through interconnected pores 117 formed therein.

To assist in implanting the orthopaedic implant 100, an opening 124 can be formed through another surface 126 of the implant body 102 to the cavity 108. The opening 124 can be any size or shape that allows for an insertion tool (not shown) to be placed within the opening 124 to help steady and position the orthopaedic implant 100 during implantation. The load bearing member 114 can partially extend into the opening 124, another material can be held in the opening 124, or the opening 124 can provide a clear path to the load bearing member 114 held in the cavity 108. In a similar manner, one or more protrusions 128 can be placed adjacent to the opening 124 that are shaped to interact with the insertion tool and provide a more stable connection between the orthopaedic implant 100 and the insertion tool. The opening 124 and protrusion(s) 128 can also be configured so that a removal tool (not shown), rather than an insertion tool, can interact with the opening 124 and protrusion(s) 128 to remove the orthopaedic implant 100 from a patient following implantation, if necessary.

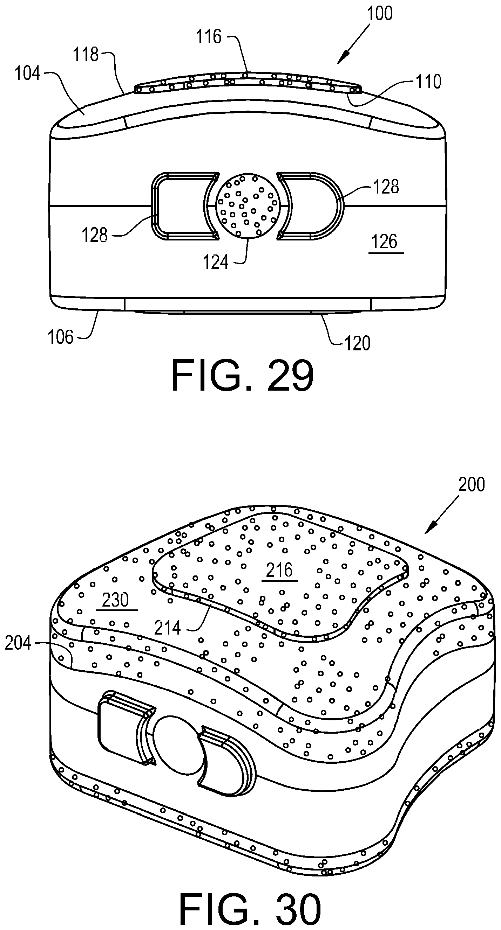

Referring now to Figs. 30-31, another embodiment of an orthopaedic implant 200 is shown that is configured similarly to orthopaedic implant 100 previously described. For brevity of description, all features of orthopaedic implant 200 that are analogous to features of orthopaedic implant 100 are numbered similarly but raised by 100. As can be seen, the first surface 204 of the implant body 202 is covered by an ingrowth material 230, shown as a porous endplate. The ingrowth material 230 can cover all or part of the first surface 204 to encourage ingrowth of surrounding tissues into the ingrowth material 230 following implantation and provide good integration of the orthopaedic implant 200. The ingrowth material 230 can be formed of any material that encourages ingrowth of a desired body tissue into the ingrowth material 230. A non-exhaustive list of contemplated materials includes porous titanium, tantalum, hydroxyapatite, tricalcium phosphate, PEEK, PAEK, polymethyl methacrylate (PMMA), polylactic acid (PLA), and polyglycolic acid (PGA), but it should be understood that many other types of materials can be used as the ingrowth material 230. Since the load bearing member 214 will initially bear the brunt of the load from surrounding anatomic structures, the ingrowth material 230 can be formed of a lower strength material, with a higher porosity than the load bearing member 214, or both. For example, the load bearing member 214 can be formed of a reinforced PEEK material that has a porosity of 60% and the ingrowth material 230 can be formed of a PEEK material that has a porosity of 80%. This allows for orthopaedic implant 200 to have a higher strength material of the load bearing member 214 initially bear the brunt of the load from surrounding anatomic structures while a higher porosity material of the ingrowth material 230 allows for better tissue ingrowth to fixate the orthopaedic implant 200.

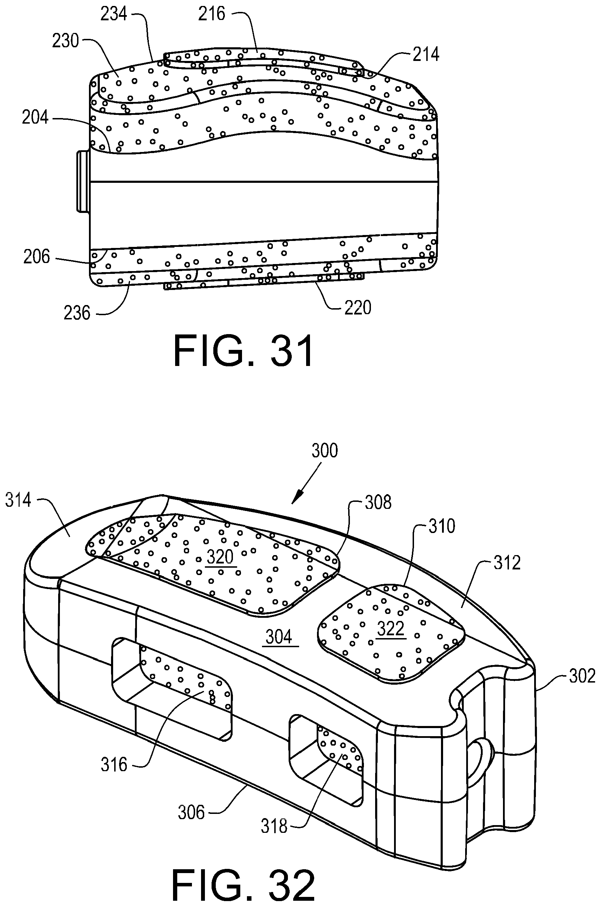

As shown in Fig. 31, the ingrowth material 230 has an ingrowth peak 234, which is the highest point of the ingrowth material 230 relative to a ground surface when the implant body 202 rests its second surface 206 on the ground surface. The first contact surface 216 of the load bearing member 214 extends out of the cavity 208 formed in the implant body 202 past the ingrowth peak 234, so that the first contact surface 216 can bear load from an anatomic structure following implantation and isolate the ingrowth material 230 from initially bearing load from the anatomic structure. The orthopaedic implant 200 can have a second ingrowth material 236 covering all or part of the second surface 206 of the implant body 202 and the load bearing member 214 can have a second contact surface 220 extending past the second ingrowth material 236 similarly to how the first ingrowth material 230 extends past the ingrowth peak 234 of the ingrowth material 230. In this sense, the ingrowth materials 230, 236 have surfaces that are analogous to the first and second surfaces 104, 106 of orthopaedic implant 100 and which the load bearing member 214 extends past.

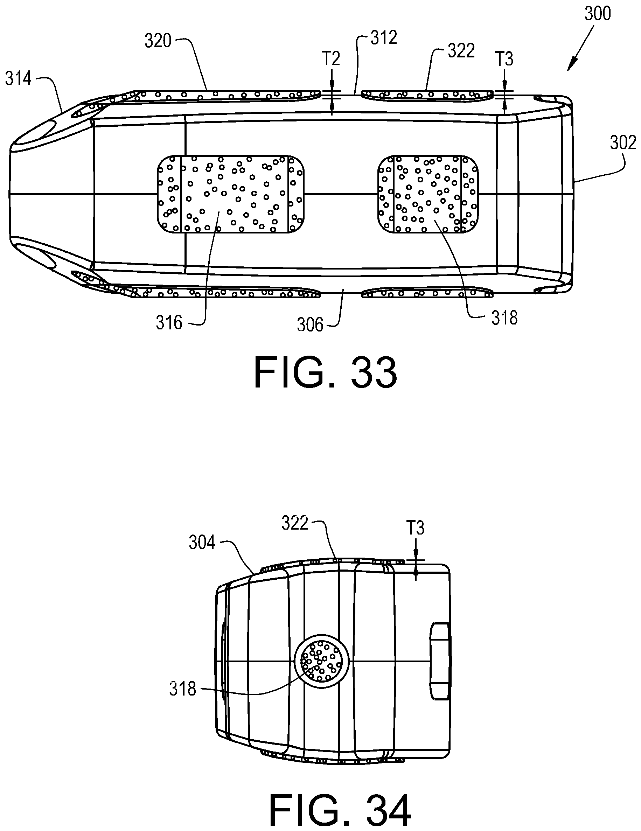

Referring now to Figs. 32-34, another embodiment of an orthopaedic implant 300 according to the present invention is shown that includes an implant body 302 configured to be used as a lumbar cage. The implant body 302 is comprised of a substantially non-porous material and has a first surface 304; a second surface 306 opposite the first surface 304; a first cavity 308 formed through the first surface 304 and second surface 306; and a second cavity 310 formed through the first surface 304 and second surface 306. As can be seen, the implant body 302 has a planar portion 312 that is flat and a curved portion 314 that has a sloped curvature. The cavities 308, 310 can be formed through the first and second surface 304, 306 all or partially within either the planar portion 312 or curved portion 314. A first load bearing member 316 is held within the first cavity 308 and a second load bearing member 318 is held within the second cavity 310. The first load bearing member 316 has a first contact surface 320 and the second load bearing member 318 has a third contact surface 322 that each extend out of their respective cavity 308, 310 past the plane of the planar portion 312, so that the contact surfaces 320, 322 can bear load from surrounding anatomic features following implantation. The load bearing members 316, 318 and their contact surfaces 320, 322 can be configured similarly to previously described load bearing members 114, 214, and even though the load bearing members 316, 318 are shown as having different sizes and total volumes, their size and total volume could be equal. The contact surfaces 320, 322 each define a respective thickness T2, T3 relative to the planar portion 312 of the first surface 304. The thicknesses T2, T3 of the contact surfaces 320, 322 can be equal to each other or could be different to provide different load bearing characteristics. For example, it may be desirable to provide load bearing member 316 with a thicker contact surface 320 than the contact surface 322 of load bearing member 318 due to the larger overall volume of load bearing member 316, in which case T2 would be greater than T3. It is also contemplated that the load bearing members 316 and 318 can be formed of different materials, have differing porosities, or be otherwise configured differently from one another to produce a desired healing effect.

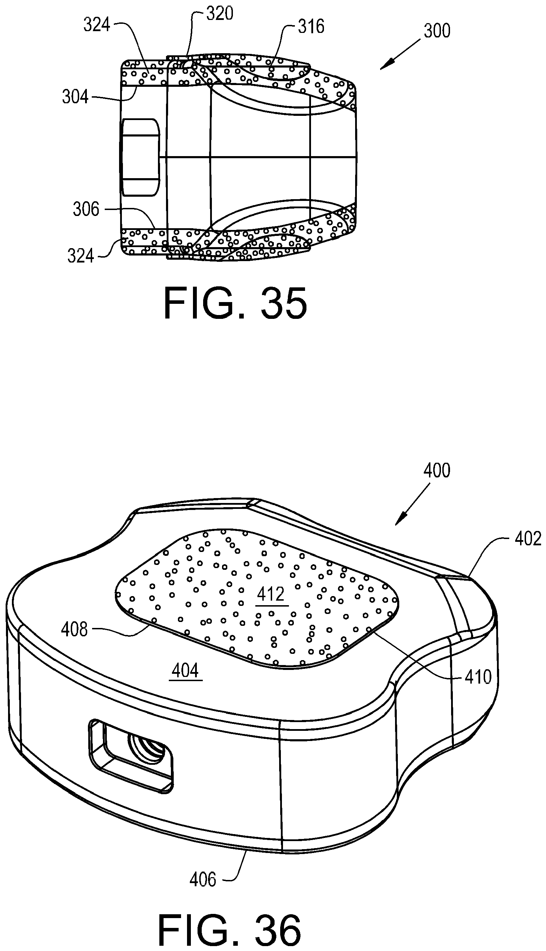

Referring now to Fig. 35, the orthopaedic implant 300 shown in Figs. 32-34 is shown with ingrowth material 324 covering the first and second surfaces 304, 306 of the implant body 302. The ingrowth material 324 can be configured in an analogous manner to previously described ingrowth material 230.

Referring now to Figs. 36-37, another embodiment of an orthopaedic implant 400 according to the present invention is shown. The orthopaedic implant 400 includes an implant body 402, configured as an anterior lumbar interbody fusion cage, comprising a substantially non-porous material having a first surface 404, a second surface 406 opposite the first surface 404, and a cavity 408 that extends through the first surface 404 and second surface 406. As can be seen, the first surface 404 is a sloped planar surface that slopes downward from a front of the implant body 402 toward a back of the implant body 402. It should be appreciated that the slope of the first surface 404 can be adjusted, as desired, to provide a variety of shapes for the implant body 402 that are suitable for different surgical procedures.

A load bearing member 410 comprising a substantially porous material is held within the cavity 408. The load bearing member 410 has a first contact surface 412 that extends out of the cavity 408 and is proud of a portion of the first surface 404 to which the first contact surface 412 is immediately adjacent. Put another way, the first contact surface 412 outwardly projects from the cavity 408 so that it will contact surrounding anatomic features when the orthopaedic implant 400 is implanted and isolate portions of the first surface 404 immediately adjacent to the cavity 408 from initially bearing load from the surrounding anatomic features. Since the first surface 404 is sloped, the first contact surface 412 does not necessarily extend past a peak of the first surface 404, as previously described first contact surfaces do. However, in all other aspects, load bearing member 410 and first contact surface 412 can be configured similarly to previously described load bearing members and contact surfaces.

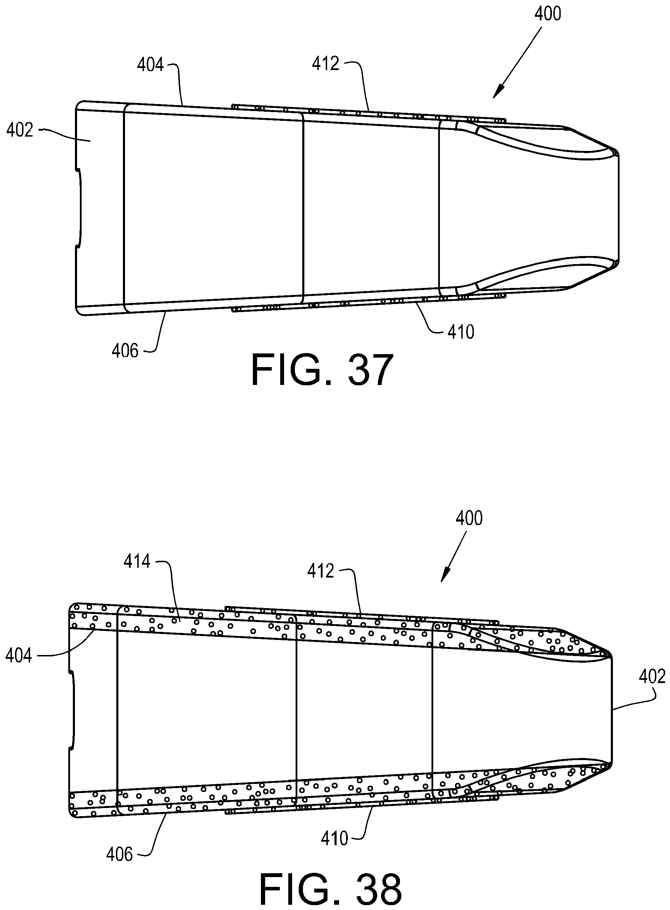

Referring now to Fig. 38, the orthopaedic implant 400 shown in Figs. 36-37 is shown with an ingrowth material 414 covering the first surface 404 of the implant body 402. The ingrowth material 414 can be configured similarly to previously described ingrowth materials. As can be seen, the load bearing member 410 is proud of a portion of the ingrowth material 414 similarly to how the load bearing member 410 shown in Figs. 36-37 is proud of a portion of the first surface 404.

While this invention has been described with respect to at least one embodiment, the present invention can be further modified within the scope of this disclosure. This application is therefore intended to cover any variations, uses, or adaptations of the invention using its general principles. Further, this application is intended to cover such departures from the present disclosure as come within known or customary practice in the art to which this invention pertains and which fall within the limits of the appended claims.

## Claims

1. An orthopaedic implant (100), comprising:
an implant body (102) having a first surface (104), a second surface (106) opposite said first surface (104), and a cavity (108) formed therein that extends through said first surface (104) and said second surface (106), said implant body being substantially non-porous;
and
a load bearing member (114) comprising a substantially porous material held within said cavity (108), said load bearing member (114) having a first contact surface (116) extending out of said cavity (108) and being proud of a portion of said first surface (104), wherein said substantially non-porous implant body (102) is a unibody and surrounds an entire periphery of at least a portion of the load bearing member (114) and the load bearing member (114) comprises a metal or a polymer,
wherein
said load bearing member (114) comprises at least one of polyether ether ketone (PEEK), tantalum, and titanium.
and/or
has a total volume and said interconnecting pores in aggregate occupy at least 60%, preferably 80% of said total volume,
and
said load bearing member (114) has a second contact surface (120) opposite said first contact surface (116), said load bearing member (114) having interconnecting pores extending from said first contact surface (116) to said second contact surface (120),
**characterized in that**
said thickness of said first contact surface (116) relative to said first surface (104) is constant throughout said first contact surface (116).

2. The orthopaedic implant according to claim 1 , wherein said second surface (106) of said implant body (102) has a peak (122), said second contact surface (120) extending out of said cavity past said peak (122).

3. The orthopaedic implant according to at least one of the claims 1 to 2,
wherein said implant body (120) has a third surface (126) with an opening formed therethrough to said cavity (108).

4. The orthopaedic implant according to claim 3, further including a protrusion (128) formed adjacent to said opening that extends away from said third surface (126).

5. The orthopaedic implant according to at least one of the claims 1 to 4,
wherein said first surface (104) defines a first surface area and said first contact surface (116) defines a contact surface area, said first surface area (104) and said contact surface (116) area together defining a total surface area, said contact surface area being at least 40% of said total surface area.

6. The orthopaedic implant according to at least one of the claims 1 to 5, further including an ingrowth material covering at least a portion of said first surface (104) of said implant body (102) and having an ingrowth peak (234), said first contact surface (116) of said load bearing member extending out of said cavity past said ingrowth peak (234).

7. The orthopaedic implant according to at least one of the claims 1 to 6,
wherein said load bearing member (114) substantially fills said cavity and/or
said first contact surface (116) is a planar surface.

8. The orthopaedic implant according to at least one of the claim 1 to 7,
wherein said first contact surface (116) defines a thickness extending past said first surface.

9. The orthopaedic implant according to at least one of the claims 1 to 8,
wherein said implant body comprises at least one of PEEK, reinforced PEEK, titanium, stainless steel, cobalt chrome, and ultra-high molecular weight polyethylene.

10. The orthopaedic implant according to at least one of the claims 1 to 9,
wherein said implant body (102) includes a second cavity formed therein that extends through said first surface (104) and said second surface (106).

11. The orthopaedic implant according to claim 10, further comprising a second load bearing member (114) comprising a substantially porous material held within said second cavity, said load bearing member (114) having a third contact surface, said third contact surface of said second load bearing member (114) extending out of said second cavity past a first peak of said first surface.

12. The orthopaedic implant according to claim 11, wherein said first contact surface (320) defines a first thickness relative to said first surface (304) and said third contact surface (322) defines a second thickness relative to said first surface (304), said second thickness being at least equal to said first thickness, preferably said second thickness is greater than said first thickness.

## Patentansprüche

1. Orthopädisches Implantat (100), umfassend:
einen Implantatkörper (102), der eine erste Oberfläche (104), eine zweite Oberfläche (106) gegenüber der ersten Oberfläche (104) und einen darin gebildeten Hohlraum (108) hat, der sich durch die erste Oberfläche (104) und die zweite Oberfläche (106) erstreckt, wobei der Implantatkörper im Wesentlichen nichtporös ist;
und
ein lasttragendes Element (114), das ein im Wesentlichen poröses Material umfasst, das im Hohlraum (108) gehalten wird, wobei das lasttragende Element (114) eine erste Kontaktfläche (116) hat, die sich aus dem Hohlraum (108) heraus erstreckt und aus einem Abschnitt der ersten Oberfläche (104) hervorragt, wobei der im Wesentlichen nichtporöse Implantatkörper (102) einteilig ist und einen kompletten Umfang mindestens eines Abschnitts des lasttragenden Elements (114) umgibt und das lasttragende Element (114) ein Metall oder ein Polymer umfasst,
wobei
das lasttragende Element (114) mindestens eines von Polyetheretherketon (PEEK), Tantal und Titan umfasst,
und/oder
ein Gesamtvolumen hat und die verbindenden Poren im Ganzen mindestens 60 %, bevorzugt 80 % des Gesamtvolumens einnehmen,
und
das lasttragende Element (114) eine zweite Kontaktfläche (120) gegenüber der ersten Kontaktfläche (116) hat, wobei das lasttragende Element (114) verbindende Poren hat, die sich von der ersten Kontaktfläche (116) zur zweiten Kontaktfläche (120) erstrecken,
**dadurch gekennzeichnet, dass**
die Dicke der ersten Kontaktfläche (116) in Bezug auf die erste Oberfläche (104) für die gesamte erste Kontaktfläche (116) konstant ist.

2. Orthopädisches Implantat nach Anspruch 1, wobei die zweite Oberfläche (106) des Implantatkörpers (102) eine Spitze (122) hat, wobei sich die zweite Kontaktfläche (120) aus dem Hohlraum heraus an der Spitze (122) vorbei erstreckt.

3. Orthopädisches Implantat nach mindestens einem der Ansprüche 1 bis 2, wobei der Implantatkörper (120) eine dritte Oberfläche (126) mit einer dahindurch gebildeten Öffnung zum Hohlraum (108) hat.

4. Orthopädisches Implantat nach Anspruch 3, das ferner einen an die Öffnung angrenzend gebildeten Vorsprung (128) beinhaltet, der sich von der dritten Oberfläche (126) weg erstreckt.

5. Orthopädisches Implantat nach mindestens einem der Ansprüche 1 bis 4, wobei die erste Oberfläche (104) einen ersten Oberflächenbereich definiert und die erste Kontaktfläche (116) einen Kontaktflächenbereich definiert, wobei der erste Oberflächenbereich (104) und der Kontaktflächenbereich (116) gemeinsam einen Gesamtflächenbereich definieren, wobei der Kontaktflächenbereich mindestens 40 % des Gesamtflächenbereichs beträgt.

6. Orthopädisches Implantat nach mindestens einem der Ansprüche 1 bis 5, das ferner ein Einwachsmaterial beinhaltet, das mindestens einen Abschnitt der ersten Oberfläche (104) des Implantatkörpers (102) bedeckt und eine Einwachsspitze (234) hat, wobei sich die erste Kontaktfläche (116) des lasttragenden Elements aus dem Hohlraum heraus an der Einwachsspitze (234) vorbei erstreckt.

7. Orthopädisches Implantat nach mindestens einem der Ansprüche 1 bis 6,
wobei das lasttragende Element (114) im Wesentlichen den Hohlraum füllt und/oder
die erste Kontaktfläche (116) eine planare Oberfläche ist.

8. Orthopädisches Implantat nach mindestens einem der Ansprüche 1 bis 7, wobei die erste Kontaktfläche (116) eine Dicke definiert, die sich an der ersten Oberfläche vorbei erstreckt.

9. Orthopädisches Implantat nach mindestens einem der Ansprüche 1 bis 8, wobei der Implantatkörper mindestens eines von PEEK, verstärktem PEEK, Titan, Edelstahl, Kobalt-Chrom und Polyethylen mit ultrahohem Molekulargewicht umfasst.

10. Orthopädisches Implantat nach mindestens einem der Ansprüche 1 bis 9, wobei der Implantatkörper (102) einen darin gebildeten zweiten Hohlraum beinhaltet, der sich durch die erste Oberfläche (104) und die zweite Oberfläche (106) erstreckt.

11. Orthopädisches Implantat nach Anspruch 10, das ferner ein zweites lasttragendes Element (114) umfasst, das ein im Wesentlichen poröses Material umfasst, das im zweiten Hohlraum gehalten wird, wobei das lasttragende Element (114) eine dritte Kontaktfläche hat, wobei sich die dritte Kontaktfläche des zweiten lasttragendes Elements (114) aus dem zweiten Hohlraum heraus an einer ersten Spitze der ersten Oberfläche vorbei erstreckt.

12. Orthopädisches Implantat nach Anspruch 11, wobei die erste Kontaktfläche (320) eine erste Dicke in Bezug auf die erste Oberfläche (304) definiert und die dritte Kontaktfläche (322) eine zweite Dicke in Bezug auf die erste Oberfläche (304) definiert, wobei die zweite Dicke der ersten Dicke mindestens gleich ist, wobei bevorzugt die zweite Dicke größer als die erste Dicke ist.

## Revendications

1. Implant orthopédique (100) comprenant :
un corps d'implant (102) ayant une première surface (104), une deuxième surface (106) opposée à ladite première surface (104), et une cavité (108) formée à l'intérieur de celui-ci qui s'étend à travers ladite première surface (104) et ladite deuxième surface (106), ledit corps d'implant étant sensiblement non poreux;
et
un élément porteur (114) comprenant un matériau sensiblement poreux maintenu dans ladite cavité (108), ledit élément porteur (114) ayant une première surface de contact (116) s'étendant hors de ladite cavité (108) et dépassant d'une portion de ladite première surface (104), dans lequel ledit corps d'implant (102) sensiblement non poreux est un monocorps et entoure une périphérie entière d'au moins une portion de l'élément porteur (114) et l'élément porteur (114) comprend un métal ou un polymère,
dans lequel
ledit élément porteur (114) comprend au moins l'un parmi de la polyéther éther cétone (PEEK), du tantale et du titane,
et/ou
a un volume total et lesdits pores d'interconnexion occupent globalement au moins 60 %, de préférence 80 % dudit volume total,
et
ledit élément porteur (114) a une deuxième surface de contact (120) opposée à ladite première surface de contact (116), ledit élément porteur (114) ayant des pores d'interconnexion s'étendant de ladite première surface de contact (116) à ladite deuxième surface de contact (120),
**caractérisé en ce que**
ladite épaisseur de ladite première surface de contact (116) par rapport à ladite première surface (104) est constante sur toute ladite première surface de contact (116).

2. Implant orthopédique selon la revendication 1, dans lequel ladite deuxième surface (106) dudit corps d'implant (102) présente un pic (122), ladite deuxième surface de contact (120) s'étendant hors de ladite cavité au-delà dudit pic (122).

3. Implant orthopédique selon au moins l'une des revendications 1 et 2,
dans lequel ledit corps d'implant (120) a une troisième surface (126) avec une ouverture formée à travers celle-ci vers ladite cavité (108).

4. Implant orthopédique selon la revendication 3, comprenant en outre une protubérance (128) formée adjacente à ladite ouverture qui s'étend à l'écart de ladite troisième surface (126).

5. Implant orthopédique selon au moins l'une des revendications 1 à 4,
dans lequel ladite première surface (104) définit une première superficie et ladite première surface de contact (116) définit une superficie de contact, ladite première superficie (104) et ladite superficie de contact (116) définissant ensemble une superficie totale, ladite superficie de contact représentant au moins 40 % de ladite superficie totale.

6. Implant orthopédique selon au moins l'une des revendications 1 à 5, incluant en outre un matériau de croissance interne couvrant au moins une portion de ladite première surface (104) dudit corps d'implant (102) et présentant un pic de croissance interne (234), ladite première surface de contact (116) dudit élément porteur s'étendant hors de ladite cavité au-delà dudit pic de croissance interne (234).

7. Implant orthopédique selon au moins l'une des revendications 1 à 6,
dans lequel ledit élément porteur (114) remplit sensiblement ladite cavité et/ou
ladite première surface de contact (116) est une surface plane.

8. Implant orthopédique selon au moins l'une des revendications 1 à 7,
dans lequel ladite première surface de contact (116) définit une épaisseur s'étendant au-delà de ladite première surface.

9. Implant orthopédique selon au moins l'une des revendications 1 à 8,
dans lequel ledit corps d'implant comprend au moins l'un parmi : de la PEEK, de la PEEK renforcée, du titane, de l'acier inoxydable, du cobalt-chrome, et du polyéthylène à poids moléculaire ultra-élevé.

10. Implant orthopédique selon au moins l'une des revendications 1 à 9,
dans lequel ledit corps d'implant (102) inclut une seconde cavité formée à l'intérieur de celui-ci qui s'étend à travers ladite première surface (104) et ladite deuxième surface (106).

11. Implant orthopédique selon la revendication 10, comprenant en outre un second élément porteur (114) comprenant un matériau sensiblement poreux maintenu dans ladite seconde cavité, ledit élément porteur (114) ayant une troisième surface de contact, ladite troisième surface de contact dudit second élément porteur (114) s'étendant hors de ladite seconde cavité au-delà d'un premier pic de ladite première surface.

12. Implant orthopédique selon la revendication 11, dans lequel ladite première surface de contact (320) définit une première épaisseur par rapport à ladite première surface (304) et ladite troisième surface de contact (322) définit une seconde épaisseur par rapport à ladite première surface (304), ladite seconde épaisseur étant au moins égale à ladite première épaisseur, de préférence ladite seconde épaisseur est supérieure à ladite première épaisseur.
